(19) Europäisches Patentamt
European Patent Office
Office européen des brevets

(11) Publication number: 0 573 837 A1

# (12) EUROPEAN PATENT APPLICATION

(21) Application number: 93108358.8

(22) Date of filing: 24.05.93

(51) Int. Cl.5: C07D 239/60, C07D 405/12, A01N 43/54

(30) Priority: 04.06.92 JP 168228/92

(43) Date of publication of application: 15.12.93 Bulletin 93/50

(84) Designated Contracting States: BE CH DE ES FR GB IT LI NL

(71) Applicant: NIHON BAYER AGROCHEM K.K.
10-8, Takanawa 4-chome
Minato-ku
Tokyo 108(JP)

(72) Inventor: Goto, Toshio
214-18, Koganei,
Kokubunji-machi
Shimotsuga-gun, Tochigi(JP)
Inventor: Kitagawa, Yoshinori
1085, Ara-machi
Moka-shi, Tochigi(JP)
Inventor: Hayakawa, Hidenori
3-17-20, Ekiminami-machi
Oyama-shi, Tochigi(JP)
Inventor: Shibuya, Katsuhiko
1425-2, Hitotonoya, Oaza
Oyama-shi, Tochigi(JP)
Inventor: Watanabe, Ryo
1057-1, Inabago, Oaza
Oyama-shi, Tochigi(JP)

(74) Representative: Schumacher, Günter, Dr. et al
Bayer AG
Konzernverwaltung RP
Patentabteilung
D-51368 Leverkusen Bayerwerk (DE)

## (54) Acetal-containing pyrimidinylthioaliphatic acid derivatives useful as herbicides.

(57) This invention relates to novel acetal-containing pyrimidinylthioaliphatic acid derivatives of the formula (I)

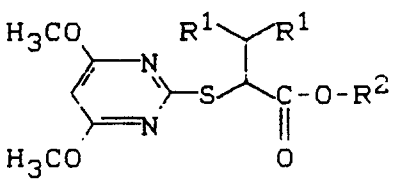

(I)

wherein

$R^1$ represents methoxy, ethoxy, methylthio, ethylthio, or the two $R^1$s may form ethylenedioxy, ethylenedithio, trimethylenedioxy or trimethylenedithio, and

$R^2$ represents hydrogen $C_{1-4}$-alkyl or benzyl,

to processes for their preparation and to their use as herbicides.

EP 0 573 837 A1

The present invention relates to novel acetal-containing pyrimidinylthioaliphatic acid derivatives, to processes for their preparation, and to their use as herbicides.

It has already been disclosed that a certain group of pyrimidinylthiocarboxylic acid derivatives are useful as herbicides (see Japanese Laid-Open Patent Application Nos. 85 262/1990 (EP-347 811/US4 968 340), 135 963/1991 (EP-409 368) and 240 777/1991).

There have now been found novel acetal-containing pyrimidinylthioaliphatic acid derivatives of the formula (I)

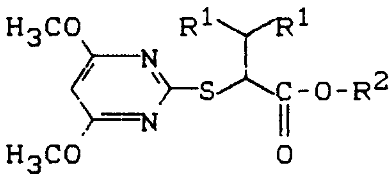

(I)

wherein

$R^1$ represents methoxy, ethoxy, methylthio, ethylthio, or the two $R^1$s may form ethylenedioxy, ethylenedithio, trimethylenedioxy or trimethylenedithio, and

$R^2$ represents hydrogen, $C_{1-4}$-alkyl or benzyl.

Acetal-containing pyrimidinylthioaliphatic acid derivatives of the formula (I) are obtained when

(a) in the case where $R^1$ is methoxy or ethoxy, and $R^2$ is not hydrogen:

compounds of the formula (II)

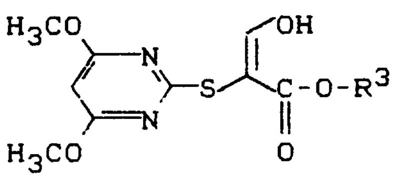

(II)

wherein

$R^3$ represents $C_{1-4}$-alkyl or benzyl,

are reacted with trimethyl orthoformate or triethyl orthoformate,

if appropriate, in the presence of acid catalyst, in the presence of inert solvents,

or

(b) in the case where the two $R^1$s are bonded together, and $R^2$ is not hydrogen:

the above compounds of the formula (II) are reacted with ethylene glycol, trimethylene glycol, 1,2-ethane dithiol or 1,3-trimethylene dithiol,

if appropriate, in the presence of acid catalyst and, if appropriate, in the presence of inert solvents,

or

(c) in the case where $R^1$ is methylthio or ethylthio, and $R^2$ is not hydrogen:

compounds of the formula (III)

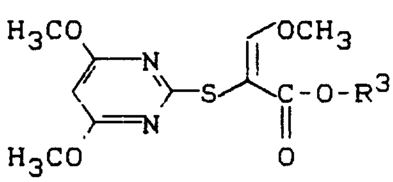

(III)

wherein

$R^3$ has the same meaning as defined above,

are reacted with methyl mercaptan or ethyl mercaptan, if appropriate, in the presence of acid catalyst and if appropriate, in the presence of inert solvents,

or

(d) in the case where $R^2$ is hydrogen:
compound of the formula (IV)

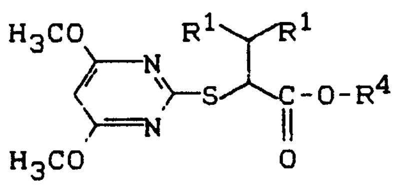

(IV)

wherein

$R^1$ has the same meaning as defined above, and
$R^4$ is $C_{1-4}$-alkyl or benzyl,

are hydrolyzed.

The novel acetal-containing pyrimidinylthioaliphatic acid derivatives of the formula (I) exhibit powerful herbicidal properties.

Surprisingly, the acetal-containing pyrimidinylthioaliphatic acid derivatives according to the invention exhibit a substantially superior selective herbicidal activity than those known from the prior art, for instance, the aforementioned Japanese Laid-Open Patent Application Nos. 85 262/1990 (EP-347 811/US 4 968 340), 135 963/1991 (EP-409 368) and 240 777/1991.

In the desired compounds and intermediate compounds represented by the above-mentioned formulas (I), (II), (III) and (IV), the $C_{1-4}$-alkyl group represents methyl ethyl n-propyl, isopropyl, or n-(iso-, sec- or tert-) butyl, preferably methyl, ethyl, n-propyl or isopropyl, more preferably methyl or ethyl.

In the compounds of the formula (I) according to the invention, in the case where $R^2$ is hydrogen, then the hydrogen may be present in a free form, or the $R^2$ may be represented in the form of a salt, including for instance, metal salt such as sodium, potassium, calcium, magnesium, aluminum, iron or cobalt salt, or organic amine salt such as methylamine-, ethylamine- or isopropylamine-addition salt.

Among the acetal-containing pyrimidinylthioaliphatic acid derivatives according to the invention of the formula (I), the preferred compounds are those in which

$R^1$ represents methoxy, ethylenedioxy or ethylenedithio, and
$R^2$ represents hydrogen or $C_{1-3}$-alkyl.

As the compounds of the formula (I) according to the invention may be mentioned:

2-(4,6-dimethoxy-2-pyrimidinylthio)-2-dioxolanyl-acetic acid ethyl ester,
2-(4,6-dimethoxy-2-pyrimidinylthio)-3,3-dimethoxy-propionic acid ethyl ester,
2-(4,6-dimethoxy-2-pyrimidinylthio)-2-dithiolanyl-acetic acid methyl ester,
2-(4,6-dimethoxy-2-pyrimidinylthio)-3,3-dimethoxy-propionic acid methyl ester,
2-(4,6-dimethoxy-2-pyrimidinylthio)-2-dithiolanyl-acetic acid ethyl ester,
2-(4,6-dimethoxy-2-pyrimidinylthio)-2-dioxolanyl-acetic acid methyl ester,
2-(4,6-dimethoxy-2-pyrimidinylthio)-2-dithiolanyl-acetic acid benzyl ester,
2-(4,6-dimethoxy-2-pyrimidinylthio)-3,3-dimethylthio-propionic acid methyl ester,
2-(4,6-dimethoxy-2-pyrimidinylthio)-2-dioxanyl-acetic acid methyl ester,
2-(4,6-dimethoxy-2-pyrimidinylthio)-2-dioxyanyl-acetic acid ethyl ester,
2-(4,6-dimethoxy-2-pyrimidinylthio)-3,3-dimethylthio-propionic acid ethyl ester,
2-(4,6-dimethoxy-2-pyrimidinylthio)-3,3-diethoxy-propionic acid methyl ester,
2-(4,6-dimethoxy-2-pyrimidinylthio)-3,3-dimethoxy-propionic acid benzyl ester,
2-(4,6-dimethoxy-2-pyrimidinylthio)-2-dithianylacetic acid methyl ester,
2-(4,6-dimethoxy-2-pyrimidinylthio)-2-dithianyl-acetic acid ethyl ester
2-(4,6-dimethoxy-2-pyrimidinylthio)-3,3-dimethoxy-propionic acid,
2-(4,6-dimethoxy-2-pyrimidinylthio)-2-dithiolanyl-acetic acid, and
2-(4,6-dimethoxy-2-pyrimidinylthio)-3,3-dimethylthio-propionic acid.

When in the process (a) for example, 3-hydroxy-2-(4,6-dimethoxy-2-pyrimidinylthio)-acrylic acid methyl ester and methyl orthoformate are used as starting materials, the course of the reaction can be represented by the following equation:

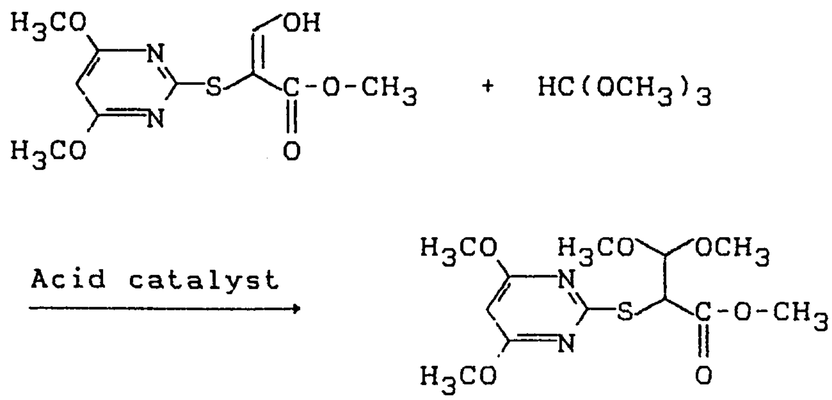


When in the process (b) for example, 3-hydroxy-2-(4,6-dimethoxy-2-pyrimidinylthio)-acrylic acid methyl ester and 1,2-ethane dithiol are used as starting materials, the course of the reaction can be represented by the following equation:

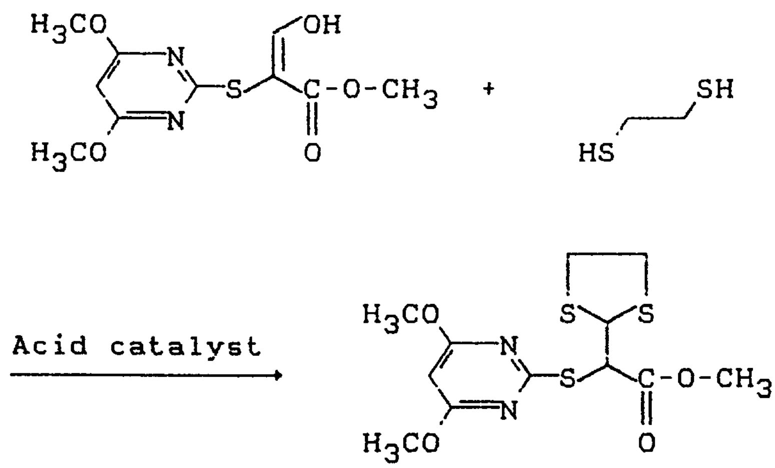


When in the process (c) for example, 3-hydroxy-2-(4,6-dimethoxy-2-pyrimidinylthio)-acrylic acid methyl ester and methyl mercaptan are used as starting materials, the course of the reaction can be represented by the following equation:

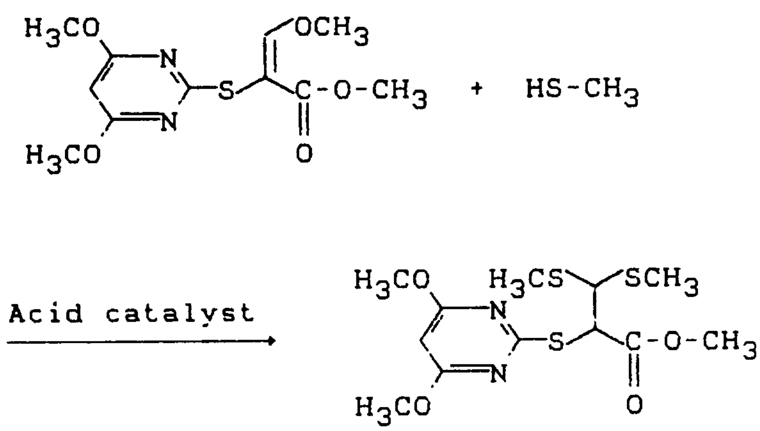


When in the process (d) for example, 2-(4,6-dimethoxy-2-pyrimidinylthio)-2-dithiolanylacetic acid methyl ester and sodium hydroxide are used as starting materials, the course of the reaction can be represented

by the following equation:

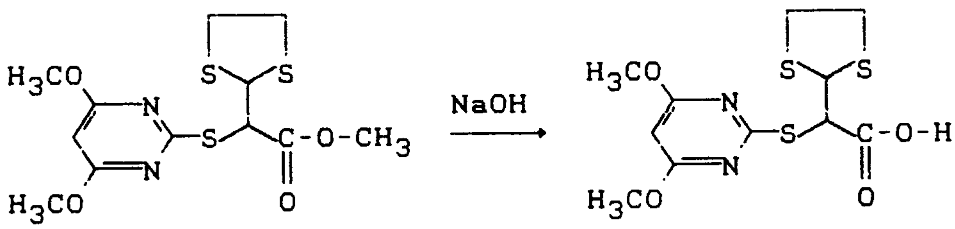


In the processes (a) and (b), the starting compounds of the formula (II) means compounds based on the above definitions of $R^3$, preferably substituents based on the above preferred definitions of $R^3$.

The compounds of the formula (II) are prepared in the conventional manner by formylating 4,6-dimethoxy-2-pyrimidinylthioacetic acid esters described, for instance, in Japanese Laid-open Patent Application No. 124 224/1992.

As specific examples of the anilines of the formula (II), there may be mentioned:
3-hydroxy-2-(4,6-dimethoxy-2-pyrimidinylthio)-acrylic acid methyl ester, and
3-hydoxy-2-(4,6-dimethoxy-2-pyrimidinylthio)-acrylic acid ethyl ester.

In the processes (c), the starting compounds of the formula (III) mean compounds based on the above definitions of $R^3$, and preferably represents methyl or ethyl.

The compounds of the formula (III) are prepared in a conventional manner by methylating the compounds of the formula (II).

In the process (d) the starting compounds of the formula (IV) mean compounds based on the above definitions of $R^1$ and $R^4$, and $R^1$ preferably based on the above preferred definitions of $R^1$, and $R^4$ represents $C_{1-3}$-alkyl.

The compounds of the formula (IV) are prepared in above processes of (a), (b) and (c).

In carrying out the process (a) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; nitriles such as acetonenitrile, propionitrile and the like; alcohols such as methanol, ethanol, isopropanol, butanol, ethylene glycol and the like; esters such as ethyl acetate, amyl acetate and the like, acid amides such as dimethyl formamide (DMF), dimethyl acetamide (DMA), N-methyl-pyrrolidone, 1,3-dimethyl-2-imidazolidinone, hexamethyl-phosphoric triamide (HMPA) and the like; sulfones and sulfoxides such as dimethyl sulfoxide (DMSO, sulfolane and the like.

The process (a) according to the invention is carried out preferably in the presence of acid catalyst. As example of such acid catalyst may be mentioned: p-toluenesulfonic acid, hydrochloric acid, sulfuric acid, acidic ion exchange resins, methanesulfonic acid, Lewis acids and the like.

In the above mentioned process (a) the reaction temperature can be varied within a substantially wide range. In general, the reaction is carried out at a temperature from about 10 °C to about 200 °C, preferably from about 40 °C to about 150 °C.

Further, the reaction is carried out under normal pressure, although it is also possible to employ a higher or reduced pressure.

When the above mentioned process (a) according to the present invention is carried out, use is made, for example, an appropriate amount of trimethyl orthoformate in a diluent such as absolute methyl alcohol, for instance, per 1 mol of the compounds of the formula (II) in the presence of an acid catalyst to obtain the desired compounds.

In carrying out the process (b) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; nitriles such as acetonenitrile, propionitrile and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like.

The process (b) according to the invention is carried out preferably in the presence of acid catalyst. As example of such acid catalyst may be mentioned: p-toluenesulfonic acid, hydrochloric acid, sulfuric acid, acidic ion exchange resins, methanesulfonic acid, Lewis acids and the like.

In the above mentioned process (b), the reaction temperature can be varied within a substantially wide range. In general, the reaction is carried out at a temperature from about 40 ° C to about 200 ° C, preferably from about 70 ° C to about 130 ° C.

Further, the reaction is carried out under normal pressure, although it is also possible to employ a higher or reduced pressure.

When the above mentioned process (b) according to the present invention is carried out, use is made, for example, 1 to 3 mol of ethylene dithiol in a diluent such as toluene, for instance, per 1 mol of the compounds of the formula (II) in the presence of an acid catalyst to obtain the desired compounds.

In carrying out the process (c) mentioned above, use may be made, as suitable diluent, of any inert solvent.

Examples of such diluents are aliphatic cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; nitriles such as acetonenitrile, propionitrile and the like; esters such as ethyl acetate, amyl acetate and the like, acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like.

The process (c) according to the invention is carried out preferably in the presence of an acid catalyst. As example of such an acid catalyst may be mentioned: p-toluenesulfonic acid, hydrochloric acid, sulfuric acid, acidic ion exchange resins, methanesulfonic acid, Lewis acids and the like.

In the above mentioned process (c), the reaction temperature can be varied within a substantially wide range. In general, the reaction is carried out at a temperature from about -30 ° C to about 200 ° C, preferably from about -10 ° C to about 130 ° C.

Further, the reaction is carried out under normal pressure, although it is also possible to employ a higher or reduced pressure.

When the above mentioned process (c) according to the present invention is carried out, use is made, for example, 1 to 3 mol of ethyl mercaptan in a diluent such as toluene, for instance, per 1 mol of the compounds of the formula (III) in the presence of an acid catalyst to obtain the desired compounds.

In carrying out the process (d) as mentioned above, use may be made, as suitable diluent, of any inert solvent.

Examples of such diluents are water; ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; alcohols such as methanol, ethanol, isopropanol, butanol, ethylene glycol and the like; acid amides such as dimethyl formamide (DMF), dimethyl acetamide (DMA), N-methyl-pyrrolidone, 1,3-dimethyl-2-imidazolidinone, hexamethyl-phosphoric triamide (HMPA) and the like; sulfones and sulfoxides such as dimethyl sulfoxide (DMSO), sulfolane and the like.

The process (d) according to the invention is carried out preferably in the presence of acid binder and as example of such acid binder may be mentioned inorganic bases including hydroxide, carbonate and bicarbonate of alkali metals, such as sodium hydrogen carbonate, potassium hydrogen carbonate, sodium carbonate, potassium carbonate, lithium hydroxide, sodium hydroxide, potassium hydroxide, calcium hydroxide and the like, and organic bases including tertiary amines, dialkylaminoanilines and pyridines such as, triethylamine, 1,1,4,4-tetramethylenediamine (TMEDA), N,N-dimethylaniline, N,N-diethylaniline, pyridine, 4-dimethylamino pyridine (DMAP), 1,4-diazabicyclo-[2,2,2]octane (DABCO), 1,8-diazabicyclo[5,4,0]-undec-7-ene (DBU) and the like.

In the above mentioned process (d), the reaction temperature can be varied within a substantially wide range. In general, the reaction is carried out at a temperature from about -10 ° C to about 150 ° C, preferably from about 10 ° C to about 80 ° C.

Further, the reaction is carried out under normal pressure, although it is also possible to employ a higher or reduced pressure.

When the above mentioned process (d) according to the present invention is carried out, use is made, for example, an appropriate amount of the compounds of the formula (IV), are subjected to a hydrolysis reaction in a diluent such as an alcohol, for example, in the presence of a base to obtain the desired compounds.

The active compounds according to the invention can be used as defoliants, desiccants, agents for destroying broad-leaved plants and, especially, as weedkillers. By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Licopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable , depending on the concentration, for the total combating of weeds, for example on industrial terrain and rail tracks, and on paths and squares with or without tree plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc. The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.001 and 4 kg of active compound per hectare of soil surface, preferably between 0.01 and 2 kg per ha.

The preparation and use of the active compounds according to the invention can be seen from the following examples.

Preparation Examples:

Example 1

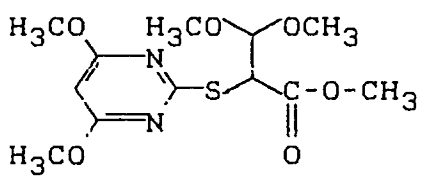

(Compound No. 1)

A mixture of 3-hydroxy-2-(4,6-dimethoxy-2-pyrimidinyl-thio)-acrylic acid methyl ester (5.0 g), absolute methanol (30 ml), methyl orthoformate (30 ml) and a catalytic amount of p-toluenesulfonic acid was heated under reflux for 3 hours. After cooling, the solvent was distilled off, and the residue was admixed with ethyl acetate and washed with an aqueous solution of sodium bicarbonate and with water. After drying over anhydrous sodium sulfate, the solvent was distilled off under reduced pressure. The resulting crude product was refined by means of a column chromatography employing hexane-ethyl acetate as the solvent, to give 2-(4,6-dimethoxy-2-pyrimidinylthio)-3,3-dimethoxy-propionic acid methyl ester (4.5 g). $n_D^{20}$ 1.5239.

Example 2

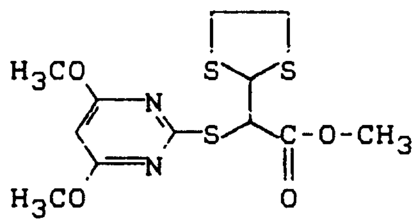

(Compound No. 4)

A mixture of 3-hydroxy-2-(4,6-dimethoxy-2-pyrimidinyl-thio)-acrylic acid methyl ester (3.0 g), ethylene dithiol (3.0 g), a catalytic amount of p-toluenesulfonic acid and toluene (30 ml) was heated under reflux for 5 hours. After cooling, the reaction mixture was washed with a 10% aqueous solution of sodium hydroxide, a 2% solution of hydrochloric acid, an aqueous solution of sodium bicarbonate and water in this order, and dried over anhydrous sodium sulfate, and the crude product thus obtained was refined by means of a column chromatography employing hexane-ethyl acetate as the solvent, to give 2-(4,6-dimethoxy-2-pyrimidinylthio)-2-dithiol-anylacetic acid methyl ester (0.5 g). $n_D^{20}$ 1.6207.

Following shows the compounds of the invention which may be obtained by the same method as above preparation examples.

Compound No. 2: 2-(4,6-dimethoxy-2-pyrimindinylthio)-3,3-dimethoxypropionicacid ethyl ester. ($n_D^{20}$ 1.5180)

Compound No. 3: 2-(4,6-dimethoxy-2-pyrimidinylthio)-3,3-diethoxypropionic acid methyl ester. ($n_D^{20}$ 1.5090)

Biological test:

Example 3

Post-emergence foliage application on upland weeds

Formulation of Active Compounds

Carrier : 5 parts by weight of acetone
Emulsifier: 1 part by weight of benzyloxy polyglycol ether

To produce a suitable formulation of each of the active compounds, 1 part by weight of the active compound was mixed with stated amount of carrier and with the stated amount of emulsifier, and the resulting emulsifiable concentrate was diluted with water to the desired concentration.

Test Procedures

In a greenhouse, a number of test pots each having an area of 250 $cm^2$ were charged with soil taken out from a cultivated field. Onto the soil surface in the respective test pots were sown the seeds of redroot pigweed (Amaranthus retroflexus), barnyard grass (Echninochloa crus-gall) and foxtail (Setaria viridis), respectively, followed by soil covering thereon. Thereafter, predetermined dosages of the active compound formulations mentioned above were uniformly sprayed onto the soil surface of the respective test pots.

Four weeks after the application of the active compound formulations, the degrees of herbicidal effect on the weeds were determined.

It was observed that, when used in an amount of 1 kg/ha, the compound Nos. 1, 2, 3 and 4 according to the invention exhibited a herbicidal effect of 100% against redroot pigweed barnyard grass and foxtail.

Example 4

Post-emergence foliage treatment on upland weeds

Test Procedures

In a greenhouse, a number of test pots each having an area of 250 $cm^2$ were charged with soil taken out from a cultivated field. Seeds of barnyard grass (Echinochloa crus-gall), foxtail (Setaria viridis), polygonum (Polygonum Convolvulus) and redroot pigweed (Amaranthus retrof lexus) respectively, were sown onto the soil surface in the respective test pots, followed by cultivation for ten days. When the test weeds were generally in the two-leaves stage, predetermined dosages of the active compound formulations prepared as in example 3 mentioned above were uniformly sprayed onto the foliage portions of the test weeds in the respective test pots.

Three weeks after the application of the active compound formulations, the degrees of herbicidal effect on the weeds are determined.

In this test, it was obtained that when used in an amount of 1 kg/ha, the compound No. 1 according to the invention, for instance, exhibited a herbicidal effect of 100% against barnyard grass, foxtail, polygonum and redroot pigweed.

## Claims

**1.** Acetal-containing pyrimidinylthioaliphatic acid derivatives of the formula (I)

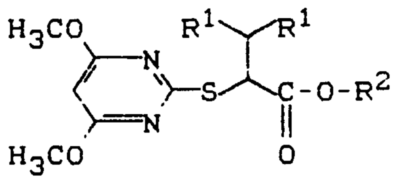

(I)

wherein

$R^1$ represents methoxy, ethoxy, methylthio, ethylthio, or the two $R^1$s may form ethylenedioxy, ethylenedithio, trimethylenedioxy or trimethylenedithio, and

$R^2$ represents hydrogen, $C_{1-4}$-alkyl or benzyl.

**2.** The compounds of the general formula (I) according to claim 1 wherein

$R^1$ represents methoxy, or the two $R^1$s may form ethylenedioxy or ethylendithio, and

$R^2$ represents hydrogen or $C_{1-3}$ alkyl.

**3.** The compounds of the general formula (I) according to claim 1 wherein such compounds are 2-(4,6-dimethoxy-2-pyrimidinylthio)-3,3-dimethoxy-propionic acid methyl ester of the following formula:

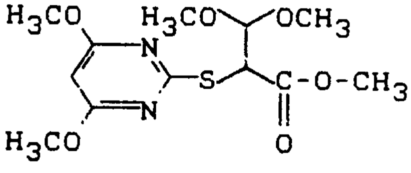

2-(4,6-dimethoxy-2-pyrimidinylthio)-3,3-dimethoxy-propionic acid ethyl ester of the following formula:

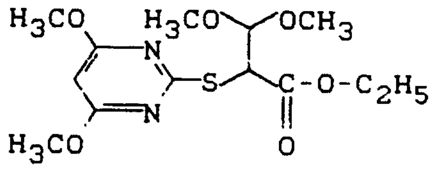

2-(4,6-dimethoxy-2-pyrimidinylthio)-3,3-diethoxy-propionic acid methyl ester of the following formula:

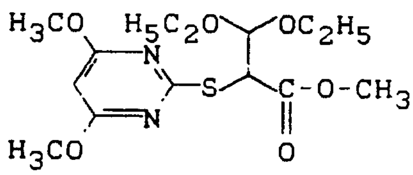

2-(4,6-dimethoxy-2-pyrimidinylthio)-2-dithiolanylacetic acid methyl ester of the following formula:

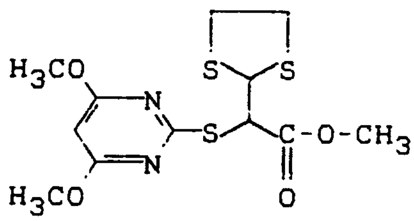

**4.** Process for the preparation of acetal-containing pyrimidinylthioaliphatic acid derivatives of the formula (I)

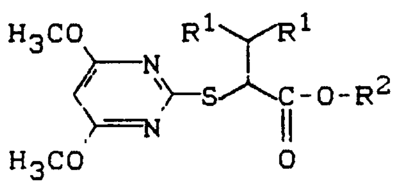

(I)

wherein

$R^1$ represents methoxy, ethoxy, methylthio, ethylthio, or the two $R^1$s may form ethylenedioxy, ethylenedithio, trimethylenedioxy or trimethylenedithio, and

$R^2$ represents hydrogen, $C_{1-4}$-alkyl or benzyl,

characterized in that

(a) in the case where $R^1$ is methoxy or ethoxy, and $R^2$ is not hydrogen:

compounds of the formula (II)

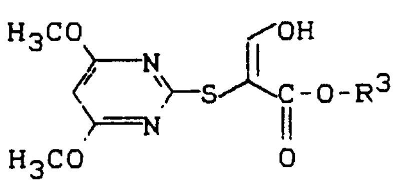

(II)

wherein

$R^3$ represents $C_{1-4}$-alkyl or benzyl,

are reacted with trimethyl orthoformate or triethyl orthoformate,

if appropriate, in the presence of acid catalyst, in the presence of inert solvents,

or

(b) in the case where the two $R^1$s are bonded together, and $R^2$ is not hydrogen:

compounds of the formula (II) are reacted with ethylene glycol, trimethylene glycol, 1,2-ethane dithiol or 1,3-trimethylene dithiol,

if appropriate, in the presence of acid catalyst and, if appropriate, in the presence of inert solvents,

or

(c) in the case where $R^1$ is methylthio or ethylthio, and $R^2$ is not hydrogen:

compounds of the formula (III)

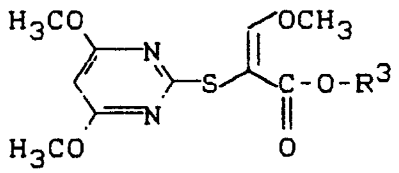

(III)

wherein

$R^3$ has the same meaning as defined above,

are reacted with methyl mercaptan or ethyl mercaptan, if appropriate, in the presence of acid catalyst and if appropriate, in the presence of inert solvents,

or

(d) in the case where $R^2$ is hydrogen:

compound of the formula (IV)

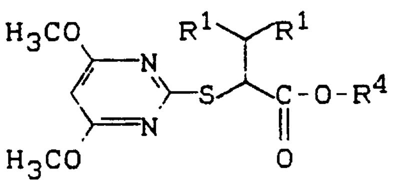

(IV)

wherein

$R^1$ has the same meaning as defined above, and

$R^4$ is $C_{1-4}$-alkyl or benzyl,

are hydrolyzed.

**5.** Herbicidal compositions, characterized in that they contain at least one acetal-containing pyrimidinylthioaliphatic acid derivative of the formula (I).

**6.** Process for combating weeds, characterized in that acetal-containing pyrimidinylthioaliphatic acid derivatives of the formula (I) are allowed to act on weeds and/or their habitat.

**7.** Use of acetal-containing pyrimidinylthioaliphatic acid derivatives of the formula (I) for combating weeds.

**8.** Process for the preparation of herbicidal compositions, characterized in that acetal-containing pyrimidinylthioaliphatic acid derivatives of the formula (I) are mixed with extenders and/or surface active agents.

European Patent Office

# EUROPEAN SEARCH REPORT

Application Number

EP 93 10 8358

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 347 811 (KUMIAI CHEMICAL INDUSTRY)<br>* page 1 - page 9; claims; table 1 *<br>--- | 1,2,4-8 | C07D239/60<br>C07D405/12<br>A01N43/54 |
| A | EP-A-0 481 512 (UBE INDUSTRIES)<br>* claims *<br>----- | 1,5-8 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 30 AUGUST 1993 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)